(19) Europäisches Patentamt
European Patent Office
Office européen des brevets

(11) Veröffentlichungsnummer: **0 259 814**
**B1**

# (12) EUROPÄISCHE PATENTSCHRIFT

(45) Veröffentlichungstag der Patentschrift: **13.12.89**

(51) Int. Cl.⁴: **C07D 309/04**, C07D 309/06, C07D 309/10, C07D 407/04, C07D 405/04, C07D 411/04

(21) Anmeldenummer: **87113021.7**

(22) Anmeldetag: **05.09.87**

(54) **Verfahren zur Herstellung von 4-Formyltetrahydropyranen und neue 4-Formyltetrahydropyrane.**

(30) Priorität: **09.09.86 DE 3630614**

(43) Veröffentlichungstag der Anmeldung: **16.03.88 Patentblatt 88/11**

(45) Bekanntmachung des Hinweises auf die Patenterteilung: **13.12.89 Patentblatt 89/50**

(84) Benannte Vertragsstaaten: **AT BE CH DE FR GB IT LI NL**

(56) Entgegenhaltungen:
**DE-A- 3 536 956**

**CHEMICAL ABSTRACTS, Band 87, Nr. 19, 7. November 1977, Columbus, Ohio, USA KUROYAN R.A.; ARUTYUNYAN N.S.; MINASYAN S.A.; "New method of synthesizing 4-aldehydes of tetrahydrothiopyran and tetrahydrothiopyran series" Seite 571, Spalte 2, Zusammenfassung Nr. 151 967tEM. INTERFACIAL ELECTROCHEM. 1982, 140(2), 329-46IM. ACTA 1978, 26(1), L13-L14 000**
**CHEMICAL ABSTRACTS, Band 76, Nr. 5, 31. Januar 1972, Columbus, Ohio, USA VARTANYAN S.A.; NORAVYAN A.S.; AVETYAN L.O. ; ZHAMAGORTSYAN V.N.; MKRTCHYAN A.P.; "Preparation of oxygen-containing six-membered heterocyclie 4-carboxaldehydes" Seite 349, Spalte 2,**

(73) Patentinhaber: **BASF Aktiengesellschaft, Carl-Bosch-Strasse 38, D-6700 Ludwigshafen(DE)**

(72) Erfinder: **Spiegler, Wolfgang, Dr., Westpreussenstrasse 5, D-6520 Worms 27(DE)**
Erfinder: **Hoelderich, Wolfgang, Dr., Mannheimer Strasse 18c, D-6710 Frankenthal(DE)**
Erfinder: **Goetz, Norbert, Dr., Schoefferstrasse 25, D-6520 Worms 1(DE)**
Erfinder: **Hupfer, Leopold, Dr., Walterhoehe 3, D-6701 Friedelsheim(DE)**
Erfinder: **Wild, Jochen, Dr., An der Marlach 7, D-6705 Deidesheim(DE)**

(56) Entgegenhaltungen: (Fortsetzung)
**Zusammenfassung Nr. 25 029yLECTROCHEM. 1982, 140(2), 329-46IM. ACTA 1978, 26(1), L13-L14 000**

EP 0 259 814 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von 4-Formyltetrahydropyranen, sowie neue 4-Formyltetrahydropyrane.

4-Formyltetrahydropyrane und einige seiner Alkylderivate sind bekannt und werden auf folgende Weise hergestellt:

Die Umsetzung von 4-Oxotetrahydropyran mit Grignard-Reagentien aus Alkoxymethylhalogeniden und Magnesium und anschließender Hydrolyse zu 4-Formyltetrahydropyran wird in Arm. Khim. Zh. 26, 227 (1973), CA 79, 66132 g; Dokl. Vscs. Konf. Khim. Atselinena 4th, 1972, 348, CA 79, 12628e und Arm. Khim. Zh. 24, 503 (1971), CA 76, 25029 y beschrieben.

Die Umsetzung von 4-Oxotetrahydropyran mit den Wittig-Reagentien Alkoxymethylentriphenyl-phosphorane liefert ebenfalls die entsprechenden 4-Formyltetrahydropyrane (vgl. Arm. Khim. Zh. 27, 945 (1974).

Nach der Glycidester-Methode von Darzens wurden verschiedene 4-Tetrahydropyranylidenglycidester in wäßriger Lösung in die Aldehyde überführt (vgl. Arm. Khim. Zh. 30, 516, (1977), CA 87, 151967 t; Sint. Geterotsikl. Soedin 1979, 25, CA 94, 30479 w; Arm. Khim. Zh. 36, 597 (1983), CA 100, 68212 j; Arm. Khim. Zh. 25, 173 (1972), CA 77, 48187 k; SU-A- 550 389, CA 87, 23051 c).

Durch Destillation von Spiro-1,6-dioxa-[2,5]-octan über Zinkchlorid wurde 4-Formyltetrahydropyran, vgl. Chem. Ber. 91, 1589 (1958), erhalten. 4-Formyltetrahydropyran wurde außerdem durch Rosemund-Reduktion aus Tetrahydropyran-4-carbonsäurechlorid sowie durch Umlagerung und anschließende katalytische Hydrierung aus 4,8-Dioxa-bicyclo[5,1,0]-octa-2,5-dien hergestellt, wie in Angew. Chemie 86, 742 (1974) beschrieben ist.

Die genannten Synthesen besitzen aber eine Reihe von Nachteilen. Die Herstellung von Alkoxymethyl-magnesiumhalogenid und Alkoxymethylen-triphenylphosphan erfordert den Einsatz von Halogenmethylethern. Wegen ihrer toxischen Eigenschaften sind dies schwer zu handhabende Substanzen, deren Einsatz - wenn technisch möglich - umgangen werden sollte. Die Glycidester-Methode liefert Ausbeuten von maximal 70 %. Für unsubstituiertes 4-Formyltetrahydropyran beträgt die Ausbeute 36 % (siehe Vergleichsbeispiel 1). Diese schlechten Ausbeuten machen diesen Syntheseweg unwirtschaftlich. Bei der Destillation von 1,6-Dioxaspiro[2,5]-octan über Zinkchlorid ist die Umsetzung unvollständig, eine Ausbeute an 4-Formyltetrahydropyran wird nicht angegeben. Tatsächlich beträgt die Ausbeute nur etwa 42 % (siehe Vergleichsbeispiel 2). Ferner wird beschrieben, daß sich beim Stehenlassen in einem geschlossenen Gefäß eine feste Kristallmasse mit einem Schmelzpunkt von 218 - 223°C bildet. 4-Formyltetrahydropyran ist aber bei Raumtemperatur eine farblose, leicht bewegliche Flüssigkeit. Ähnliches gilt für die Rosemund-Reduktion von Tetrahydropyran-4-carbonsäurechlorid. Auch hier werden keine Ausbeuten angegeben und für das Reaktionsprodukt wird ein Schmelzpunkt von 135°C genannt, was bedeutet, daß es sich ebenfalls nicht um 4-Formyltetrahydropyran handeln kann. Bei diesen, dem 4-Formyltetrahydropyran zugeordneten Feststoffen handelt es sich vermutlich um höhermolekulare Addukte des Aldehyds, die für eine Reihe von Folgereaktionen aber nicht verwendet werden können. 4,8-Dioxa-bicyclo[5.1.0]-octa-2,5-dien ist nur schwer zugänglich und als Einsatzstoff für ein technische Synthese völlig ungeeignet.

Auch ist bekannt, daß aus substituierten 1,2-Diolen (Übersichtsartikel in Houben-Weyl, Methoden der organischen Chemie, Band E 3, 491, (1983), substituierten Oxiranen (Übersichtsartikel in Houben-Weyl, Methoden der organischen Chemie, Band E 3, 496, (1983)) und aus substituierten tert.-Butylglycidestern (Übersichtsartikel in Houben-Weyl, Methoden der organischen Chemie, Band E 3, 530, (1983) die entsprechenden Aldehyde erhalten werden.

Außerdem ist bekannt, daß sich Butylenoxid an dotierten A-Zeolithen zu 55 - 72 % Butyraldehyd, 19 - 34 % cis-trans-But-2-en-ol, 3 - 9 % Butanol und 2 % Methylethylketon umsetzen läßt, vgl. Hokkaido Dagaku Kogakubu Kenkyn Hokoku 67 (1973), 171 - 178. Die Selektivität läßt hierbei noch zu wünschen übrig. Auch läßt sich der A-Zeolith-Katalysator nach seiner Desaktivierung durch Koks nicht regenerieren, da bei den hierfür notwendigen Temperaturen von ca. 500°C die Kristallstruktur dieses Zeolithen zerstört wird.

Dies gilt auch für die Herstellung von 2-(4')-Isobutyl-phenyl-propanal aus dem 2-4'-Isobutyl-phenyl-2-methyl-oxiran an 5 Å Molekularsieben, vgl. JP-A-3031-637.

Für die Umsetzung von Propylenoxid zu Aceton oder Propionaldehyd an alkalidotierten X-Zeolithen (Waseda Daigaku Rikogaku Kenkyusho Hokoku 67 (1974), 26 - 9) ist es notwendig in Abwesenheit stark acider Zentren zu arbeiten.

Cyclododecanon wird an Ph- oder Rh-dotiertem $Al_2O_3$ aus Epoxycyclododecan erhalten (Neftekhimiya 16 (1976), 250 - 54). In dieser Arbeit wird ausdrücklich darauf hingewiesen, daß Zeolithe für diese Reaktion ungeeignet sind.

In EP-A-100 117 wird die Reaktion von Styroloxid und von am aromatischen Kern alkyl- bzw. alkoxylsubstituierten Styroloxiden an einem Titan-haltigen Zeolithen zu β-Phenylacetaldehyden in der flüssigen Phase bei 30 bis 100°C beschrieben. Der hierzu eingesetzte Katalysator muß aufwendig aus teuren hochreinen Edukten wie Tetraalkylorthosilikat, Tetraalkylorthotitanat und Tetrapropylammoniumhydroxid hergestellt werden. Hoher Umsatz wird nur erzielt, wenn die Reaktion in einem Lösungsmittel wie Methanol oder Aceton bei 30 bis 100°C in der Flüssigphase stattfindet und Verweilzeiten von 1 bis 1,5 h eingehalten werden. Dies zieht erhöhte Destillations- und Betriebskosten nach sich. Weiterhin besitzt die Reakton an Titan-haltigen Zeolithen keine Allgemeingültigkeit und ist nur bei Styroloxid und am aromatischen Kern alkylierten bzw. alkoxylierten Styroloxiden möglich.

Der Erfindung liegt die Aufgabe zugrunde, ein Verfahren zur Herstellung von neuen und bekannten 4-Formyltetrahydropyranen, sowie neue 4-Formyltetrahydropyrane zur Verfügung zu stellen.

Diese Aufgabe wird gelöst mit einem Verfahren der genannten Art zur Herstellung von 4-Formyltetrahydropyranen der allgemeinen Formel I

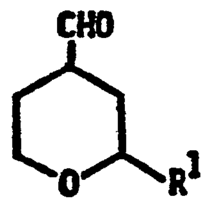

(I) ,

worin $R^1$ für Wasserstoff, $C_1$-$C_{12}$-Alkyl, $C_2$-$C_{12}$-Alkenyl, $C_1$-$C_6$-Alkoxy-substituiertes $C_1$-$C_{12}$-Alkyl, $C_4$-$C_{12}$-Cycloalkyl, gegebenenfalls substituiertes Aryl, Heteroaryl, oder Aralkyl, oder einen gegebenenfalls substituierten, gesättigten oder ungesättigten 5- oder 6-gliedrigen Ring, der gegebenenfalls durch N, O oder S unterbrochen ist, steht, das dadurch gekennzeichnet ist, daß man ein Pyranderivat der allgemeinen Formel II

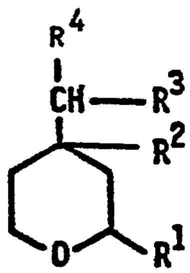

(II) ,

worin $R^1$ wie zuvor definiert ist,
$R^2$ und $R^3$ jeweils für eine OH-Gruppe stehen, oder gemeinsam ein Sauerstoffatom darstellen und dabei einen Oxiranring bilden, und
$R^4$ in dem Fall, daß $R^2$ und $R^3$ jeweils für eine OH-Gruppe stehen, Wasserstoff bedeutet und in dem Fall, daß $R^2$ und $R^3$ gemeinsam ein Sauerstoffatom darstellen, Wasserstoff oder tert.-Butyloxycarbonyl bedeutet,
bei erhöhter Temperatur mit einem Katalysator, ausgewählt unter Zeolithen, Phosphaten, Borsäure auf einem Trägermaterial und Siliciumdioxid, oder deren Mischungen, behandelt.

Die neuen 4-Formyltetrahydropyrane entsprechen der allgemeinen Formel I'

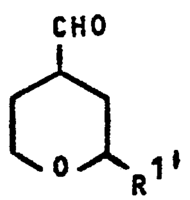

(I') ,

worin $R^{1'}$ für $C_4$-$C_{12}$-Alkyl, $C_4$-$C_{12}$-Cycloalkyl, $C_1$-$C_6$-alkoxysubstituiertes $C_1$-$C_{12}$-Alkyl, gegebenenfalls durch $C_1$-$C_6$-Alkoxy, Halogen oder Trifluormethyl substituiertes Aryl oder Heteroaryl, Aralkyl mit 1 bis 6 Kohlenstoffatomen im Alkylteil, oder einen 5- oder 6-gliedrigen, gesättigten oder ungesättigten Ring, der durch mindestens ein N, O oder S-Atom unterbrochen ist, steht.

Die als Ausgangsverbindungen der allgemeinen Formel II eingesetzten Pyranderivate entsprechen den nachstehenden allgemeinen Formeln II a, II b und II c.

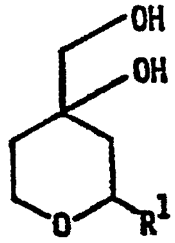 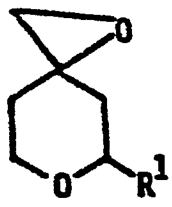 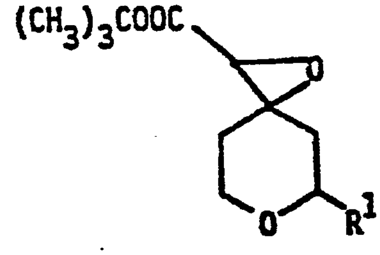

(II a) (II b) (II c)

worin $R^1$ die eingangs angegebenen Bedeutungen besitzt.

Beim erfindungsgemäßen Verfahren ist es bevorzugt, als Ausgangsverbindung der allgemeinen Formel II das 4-[1'2'-Dihydroxyethyl]tetrahydropyran oder 1,6-Dioxaspirol[2.5]octan einzusetzen.

Wenn der Rest $R^1$ in der allgemeinen Formel I für $C_1$–$C_{12}$-Alkyl steht, so bedeutet er insbesondere Methyl, Ethyl, n-Propyl, Isopropyl, n-Butyl, Isobutyl, Pentyl, Hexyl, Octyl, Decyl und Dodecyl. Die $C_1$–$C_{12}$-Alkylreste können geradkettig oder verzweigt sein.

Wenn $R^1$ für $C_2$–$C_{12}$-Alkenyl steht, so kann er beispielsweise Ethenyl, Propenyl, Butenyl oder Pentenyl bedeuten. Der $C_1$–$C_{12}$-Alkylrest kann gewünschtenfalls durch einen $C_1$–$C_6$-Alkoxyrest substituiert sein. Als Alkoxyreste kommen insbesondere in Betracht Methoxy, Ethoxy, Propoxy- und Butoxy-. Der Rest $R^1$ kann auch für $C_4$–$C_{12}$-Cycloalkyl stehen, beispielsweise für Cyclobutyl, Cyclopropyl, Cyclohexyl und Cyclooctyl und Cyclododecyl.

$R^1$ bedeutet auch gegebenenfalls substituiertes Aryl, Heteroaryl oder Aralkyl. Als Substituenten kommen hierbei die üblichen in Betracht, insbesondere $C_1$–$C_6$-Alkylreste, Halogen, insbesondere Fluor, Chlor, Brom und $C_1$–$C_6$-Alkoxy. Als Beispiele für Aryl-, Heteroaryl- oder Aralkylreste sind insbesondere zu nennen Phenyl, Naphthyl, Pyridyl, Thienyl, Furyl, Benzyl oder Phenylethyl.

$R^1$ kann auch für einen ggf. substituierten, gesättigten oder ungesättigten 5- oder 6-gliedrigen Ring stehen, der ggf. durch N, O oder S unterbrochen ist. Zu erwähnen sind hierbei insbesondere Tetrahydropyranyl, Furanyl, Morpholinyl.

In der allgemeinen Formel I' steht der Rest $R^{1'}$ für $C_4$–$C_{12}$-Alkyl, beispielsweise für Butyl, Pentyl, Hexyl, Octyl, Decyl, Dodecyl. Er kann auch für $C_4$–$C_{12}$-Cycloalkyl stehen und die im Zusammenhang mit der Erläuterung der Bedeutungsmöglichkeit des Restes $R^1$ angegebenen Bedeutungen besitzen. Dasselbe gilt für die Bedeutungsmöglichkeiten $C_1$-$C_6$-Alkoxy-subtituiertes $C_1$-$C_{12}$-Alkyl, sowie die restlichen Bedeutungsmöglichkeiten von $R^{1'}$.

Im Vergleich zu den bekannten Synthesen von 4-Formyltetrahydropyranen bietet das erfindungsgemäße Verfahren eine Reihe von Vorteilen. So wird z.B. 4-Formyltetrahydropyran erstmalig aus gut verfügbaren, billigen Einsatzstoffen in hoher Ausbeute erhalten und zwar in der monomeren Form, was für Folgereaktionen von großer Bedeutung ist. Ferner kann auf den Einsatz der schwer handhabbaren, toxischen Halogenmethyl-ether zur Herstellung der Grignard- oder Wittig-Reagentien für die Umsetzung mit 4-Oxotetrahydropyranen verzichtet werden. Die Ausbeuten bei der Umsetzung der tert.-Butylglycidester II c sind wesentlich höher als die, die bei der Herstellung von 4-Formyltetrahydropyranen aus Glycidestern bisher erzielt wurden.

Mit den im erfingungsgemäßen Verfahren eingesetzten zeolithischen Katalysatoren werden ausgezeichnete Ausbeuten, Selektivitäten und Standzeiten erzielt. Dies ist umso überraschender, wenn man berücksichtigt, daß die Reaktionen zweckmäßig bei hohen Temperaturen in der Gasphase durchgeführt und dabei die empfindlichen 4-Formyltetrahydropyrane durch die aciden Zeolithe trotz ihrer Crackeigenschaften nicht abgebaut werden.

Die erfindungsgemäß verwendeten Katalysatoren, zeichnen sich durch einfache Verfügbarkeit, hohe Aktivität und leichte Regenerierbarkeit (Anfangsaktivität wird zurückerhalten) aus. Weiterhin sind bei langen Katalysatorstandzeiten hohe Umsätze, hohe Selektivitäten und eine flexible Einsetzbarkeit des Katalysators hinsichtlich der Edukte gewährleistet.

Im erfindungsgemäßen Verfahren werden die eingangs erwähnten Nachteile der bisherigen Verfahrensweisen vermieden. Im Hinblick auf den Stand der Technik ist das Gelingen des Verfahrens umso überraschender, als man bisher nur schwach acide X-Zeolithe einsetzte bzw. Zeolithe für Umlagerungsreaktionen als ungeeignet einstufte. Daher konnte man nicht erwarten, daß gerade mit Zeolithen, die sich durch hohe Acidität sowie durch strenge Strukturparameter auszeichnen, in so weiten Grenzen und bei so großer Vielfalt der Edukte solch hervorragende Ergebnisse erhalten werden.

Vorteile des erfindungsgemäßen Verfahrens bei der Umlagerung an den erfindungsgemäßen Katalysatoren sind: Vollständiger Umsatz, keine Trennprobleme, lange Standzeiten, hohe Selektivitäten, auch sehr gute Ausbeuten bei am aromatischen Kern substituierten Ausgangsprodukten, einfache Isolierung der Endprodukte, in der Regel Weiterverwendung ohne zusätzliche Reinigung, leichte Regenerierbarkeit der Katalysatoren bei eventuell auftretender Verkokung. Ein weiterer Vorteil ist es, daß man die Reaktion in der bevorzugten Gasphase ausführen kann.

Zusammenfassend gesehen ist das neue Verfahren demnach wirtschaftlicher und technisch einfacher zu realisieren als die bekannten Synthesen und liefert 4-Formyltetrahydropyrane in höherer Ausbeute und Reinheit als bisher.

Die Ausgangsverbindungen der allgemeinen Formel II können auf herkömmlichem Weg aus den entsprechenden 4-Methylen-tetrahydropyranen durch Oxidation zu den Diolen (II a) (Khim. Geterotsikl. Soedin. 1983, 891) oder zu den Oxiranen (II b) (Izv. Akad. Nauk. SSSR, Ser. Khim. 1982, 2114) oder aus den entsprechenden 4-Oxotetrahydropyranen durch Umsetzung mit Chloressigsäure-tert.-butylester zu den Glycidestern (II c) erhalten werden. Die eingesetzten 4-Methylentetrahydropyrane werden durch Umsetzung von 3-Methyl-3-buten-1-ol mit Aldehyden der allgemeinen Formel (III)

$$R^1\text{-CHO} \quad (III),$$

worin $R^1$ wie zuvor definiert ist, wie z.B. in Arm. Khim. Zh. 29 1033, (1976), CA 87, 5757 h, beschrieben hergestellt und durch anschließende Destillation gereinigt. Das dabei anfallende Diastereomerenpaar wird ohne Trennung in die Umlagerungsreaktion zum Diastereomerengemisch der Aldehyde I eingesetzt.

Die oben genannten Verbindungen stellen eine Auswahl verwendbarer Komponenten zur Herstellung von Aldehyden der allgemeinen Formel I dar und sollen die Anwendungsbreite des erfindungsgemäßen Verfahrens für eine Vielzahl von Aldehyden der Formel I nicht einschränken.

Als Katalysatoren für die erfindungsgemäße Umwandlung werden zweckmäßig acide zeolithische Katalysatoren eingesetzt. Zeolithe sind kristalline Aluminosilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1 : 2 (siehe Ullmanns Encyclopädie d. techn. Chemie, 4. Auflage, Band 24, S. 575 (1983)). Die Elektrovalenz der Aluminium enthaltenden Tetraeder ist durch Einschluß von Kationen in den Kristall, z.B. eines Alkali- oder Wasserstoffions, ausgeglichen. Ein Kationenaustausch ist möglich. Die Räume zwischen den Tetraedern sind vor der Dehydratation durch Trocknen bzw. Calcinieren von Wassermolekeln besetzt.

In den Zeolithen können anstelle von Aluminium auch andere drei- und zweiwertige Elemente wie B, Ga, Fe, Cr, Be, As, Sb und Bi oder deren Gemische in das Gitter eingebaut werden, oder das Silicium kann durch ein vierwertiges Element wie Ge, Ti, Zr, Hf, ersetzt werden.

Als Katalysatoren kommen Zeolithe aus der Mordenit-Gruppe oder Faujasit-Gruppe wie L-Zeolith oder engporige Zeolithe vom Erionit- bzw. Chabasit-Typ in Betracht. Besonders vorteilhaft für das erfindungsgemäße Verfahren sind Zeolithe vom Pentasil-Typ. Diese Zeolithe können unterschiedliche chemische Zusammensetzung aufweisen. Es handelt sich hierbei um Alumino-, Boro-, Eisen-, Gallium-, Chrom-, Beryllium-, Arsen-, Antimon- und Wismutsilikatzeolithe oder deren Gemische sowie um Alumino-, Boro-, Gallium- und Eisengermanatzeolithe oder deren Gemische.

Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in 1,6-Hexandiamin- oder 1,3-Propandiamin- oder Triethylentetramin-Lösung mit oder insbesondere ohne Alkali- oder Erdalkalizusatz bei 100 bis 220°C unter autogenem Druck hergestellt. Auch gehören hierzu die isotaktischen Zeolithe nach DE-A-3 006 471 und EP-A-46 504. Die erhaltenen Aluminosilikatzeolithe enthalten je nach Wahl der Einsatzstoffmengen ein $SiO_2/Al_2O_3$-Verhältnis von 10 bis 40.000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200°C unter autogenem Druck synthetisiert, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit oder insbesondere ohne Alkali- oder Erdalkalizusatz zur Reaktion bringt. Auch gehören hierzu die isotaktischen Zeolithe nach DE-A-3 006 471 und EP-A-46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220°C unter autogenem Druck.

Die so hergestellten Alumino-, Boro- und Eisensilikatzeolithe können nach ihrer Isolierung, Trocknung bei 100 bis 160°C, vorzugsweise 110°C und Calcinierung bei 450 bis 550°C, vorzugsweise 500°C bis 540°C, mit einem Bindemittel im Verhältnis 90 : 10 bis 40 : 60 Gew.-% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 95 : 5, bevorzugt 75 : 25, Siliciumdioxid, bevorzugt hochdisperses $SiO_2$, Gemische aus hochdispersem $SiO_2$ und hochdispersem $Al_2O_3$ sowie Ton. Nach der Verformung werden die Extrudate oder Preßlinge bei 110°C/16 h getrocknet und bei 500°C/16 h calciniert.

Man erhält auch vorteilhaft Katalysatoren, wenn der isolierte Alumino- bzw. Borosilikatzeolith direkt nach der Trocknung verformt wird und erstmals nach der Verformung einer Calcinierung unterworfen wird. Die hergestellten Alumino- und Borsilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung, oder durch Behandlung mit Säuren, vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550°C, bevorzugt 500°C bis 540°C, zu regenerieren. Die Zeolithe erhalten dadurch ihre Anfangsaktivität zurück. Durch partielle Verkokung (pre-coke) ist es möglich, die Aktivität des Katalysators für ein Selektivitätsoptimun des gewünschten Reaktionsproduktes einzustellen.

Um eine möglichst große Selektivität, hohen Umsatz sowie lange Standzeiten zu erreichen, ist es mitunter vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert.

Zweckmäßigerweise führt man die Dotierung so durch, daß man z.B. den verformten Pentasilzeolithen in einem Steigrohr vorlegt und bei 20 bis 100°C z.B. eine wäßrige oder ammoniakalische Lösung eines Halogenids oder eines Nitrates der Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einen Nitrat oder einem Oxid der Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cs_2CO_3$ in Wasser löst. Mit dieser Lösung wird der verformte oder unverformte Zeolith eine gewisse Zeit, beispielsweise ca. 30 Minuten, getränkt. Die eventuell überstehende Lösung wird am Rotationsverdampfer von Wasser befreit. Danach wird der getränkte Zeolith bei ca. 150°C getrocknet und bei ca. 550°C calciniert. Dieser Tränkvorgang kann mehrmals hintereinander vorgenommen werden, um den gewünschten Metallgehalt einzustellen.

Auch ist es möglich, z.B. eine ammoniakalische $Pd(NO_3)_2$-Lösung herzustellen und darin den reinen pulverförmigen Zeolithen bei 40 bis 100°C unter Rühren ca. 24 h aufzuschlämmen. Nach Abfiltrieren, Trocknen bei ca. 150°C und Calcinierung bei ca. 500°C kann das so gewonnene zeolithische Material mit oder ohne Bindemittel zu Strängen, Pellets oder Wirbelgut weiterverarbeitet werden.

Ein Ionenaustausch des in der H-Form vorliegenden Zeolithen kann so vorgenommen werden, daß man den Zeolithen in Strängen oder Pellets in einer Kolonne vorlegt und darüber z.B. eine ammoniakalische $Pd(NO_3)_2$-Lösung bei leicht erhöhter Temperatur zwischen 30 und 80°C im Kreislauf 15 bis 20 h leitet. Danach wird mit Wasser ausgewaschen, bei ca. 150°C getrocknet und bei ca. 550°C calciniert.

Bei manchen metalldotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft.

Zu den verwendbaren siliciumreichen Zeolithen (molares Verhältnis $SiO_2/Al_2O_3 \geq 10$) gehören auch die bekannten ZSM-Typen sowie Ferrierit und Nu-1 sowie Silicalit®, ein Molekularsieb, ein sog. Silica Polymorph.

Weitere Katalysatoren für die Herstellung von Aldehyden der allgemeinen Formel I aus entsprechenden Epoxiden der allgemeinen Formel II sind im folgenden beschrieben:

Als Aluminiumphosphat-Katalysatoren werden für das erfindungsgemäße Verfahren insbesondere unter hydrothermalen Bedingungen synthetisierte Aluminiumphosphate eingesetzt.

Die unter hydrothermalen Bedingungen hergestellten Aluminiumphosphate sind z.B. APO-5, APO-9, APO-11, APO-12, APO-14, APO-21, APO-25 und APO-33. Synthesen dieser Verbindungen sind in EP-A-132 708, US-A-4 310 440 und US-A-4 473 663 beschrieben.

Beispielsweise das $AlPO_4$-5 (APO-5) wird synthetisiert, indem man Orthophosphorsäure mit Pseudoboehmit (Catapa® SB) in Wasser homogen mischt, zu dieser Mischung Tetrapropylammoniumhydroxid gibt und danach bei ca. 150°C 20-60 h unter autogenem Druck in einem Autoklaven umsetzt. Das abfiltrierte $AlPO_4$-5 wird getrocknet bei 100 - 160°C und calciniert bei 450 - 550°C.

$AlPO_4$-9 (APO-9) wird ebenfalls aus Orthophosphorsäure und Pseudoboehmit, aber in wäßriger DABCO-Lösung (1,4-Diazabicyclo-(2,2,2)octan) bei ca. 200°C unter autogenem Druck während 200 - 400 h synthetisiert.

Die Synthese des $AlPO_4$-21 (APO-21) erfolgt aus Orthophosphorsäure und Pseudoboehmit in wäßriger Pyrrolidon-Lösung bei 150 - 200°C unter autogenem Druck während 50 - 200 h.

Die für das erfindungsgemäße Verfahren eingesetzten Siliciumaluminiumphosphate sind z.B. SAPO-5, SAPO-11, SAPO-31 und SAPO-34. Die Synthese dieser Verbindung wird z.B. in EP-A-103 117, US-A-4 440 871 beschrieben. SAPO's werden hergestellt durch Kristallisation aus wäßriger Mischung bei 100 - 250°C und autogenem Druck während 2 h - wobei die Reaktionsmischung aus einer Silicium-, Aluminium- und Phosphorkomponente in wäßrigen aminoorganischen Lösungen umgesetzt wird.

SAPO-5 beispielsweise wird durch Mischen von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 - 200°C während 20 - 200 h unter autogenem Druck in einem Rührautoklaven erhalten. Die Trocknung des abfiltrierten Pulvers erfolgt bei 110 - 160°C und die Calcination bei 450 - 550°C.

Als Siliciumaluminiumphosphate sind z.B. ZYT-5, ZYT-6, ZYT-7, ZYT-9, ZYT-11 und ZYT-12 geeignet (JP-A-5 9217-619).

Borphosphate für das erfindungsgemäße Verfahren lassen sich beispielsweise durch Mischen und Kneten von konzentrierter Borsäure und Phosphorsäure und durch anschließende Trocknung und Calcination in Inertgas-, Luft- oder Dampfatmosphäre bei 250 - 650°C, vorzugsweise 300 - 500°C, herstellen.

Als Phosphate lassen sich auch $CePO_4$, $FePO_4$, $SrHPO_4$ und $Zr_3(PO_4)_4$ für das erfindungsgemäße Verfahren verwenden.

Als weitere Katalysatoren für das erfindungsgemäße Verfahren werden eingesetzt: Phosphorsäure oder Borsäure auf $SiO_2$-, $Al_2O_3$- oder Bims-Trägern z.B. durch Auftränken oder Versprühen aufgebracht. Ein phosphorsäurehaltiger Katalysator kann beispielsweise durch Auftränken von $H_3PO_4$ - oder $NaH_2PO_4$- oder $Na_2HPO_4$-Lösung auf $SiO_2$ und anschließende Trocknung bzw. Calcination erhalten werden. Phosphorsäure kann aber auch mit Kieselgel zusammen in einem Sprühturm versprüht werden, danach schließen sich eine Trocknung und meist eine Calcination an. Phosphorsäure kann auch in einer Imprägniermühle auf das Trägermaterial aufgesprüht werden. Auch läßt sich Siliciumdioxid als Katalysator einsetzen.

Die hier beschriebenen Katalysatoren können wahlweise als 2 bis 4 mm Stränge oder als Tabletten mit 3 bis 5 mm Durchmeser oder als Pulver mit Teilchengrößen von 0,1 - 0,5 mm oder als Wirbelkontakt eingesetzt werden.

Die für die erfindungsgemäße Umwandlung der Epoxide in der Regel gewählten Reaktionsbedingungen sind in der bevorzugten Gasphase 150 bis 500°C, vorzugsweise 200 bis 400°C, und eine Belastung WHSV = 0,1 bis 20 $h^{-1}$, bevorzugt 0,5 bis 5 $h^{-1}$ (g Epoxide/g Katalysator und Stunde). Im allgemeinen steigt der Umsatz mit steigender Temperatur stark an, während die Selektivität in einem bestimmten Temperaturbereich nur wenig zurückgeht.

Auch ist es möglich, die Reaktion in der Flüssigphase (Suspensions-, Riesel- oder Sumpffahreweise) durchzuführen.

Das Verfahren wird in der Regel bei Normaldruck oder je nach Flüchtigkeit der Ausgangsverbindung bei vermindertem oder erhöhtem Druck durchgeführt, wobei die Durchführung diskontinuierlich, aber vorzugsweise kontinuierlich erfolgen kann.

Schwerflüchtige oder feste Edukte werden in gelöster Form, z.B. in THF-, Toluol- oder Petrolether-Lösung zum Einsatz gebracht. Generell ist auch eine Verdünnung mit Lösungsmitteln wie z.B. voranstehend genannt oder Inertgasen wie $N_2$, Ar, möglich.

Nach der Umsetzung werden die entstandenen Tetrahydropyrane nach üblichen Techniken, z.B. durch Destillation aus dem Reaktionsgemisch isoliert; nichtumgesetzte Edukte werden gegebenenfalls, wenn nötig, für die erfindungsgemäße Umsetzung zurückgeführt. Eine direkte Weiterverarbeitung der Reaktionsprodukte ist aufgrund der sehr hohen Ausbeuten möglich. Bei dem erfindungsgemäßen Verfahren fallen bevorzugt die monomeren Verbindungen an.

Die erfindungsgemäßen Pyrane der allgemeinen Formel I sind wertvolle Zwischenprodukte für die Synthese von Pflanzenschutzmitteln, Pharmazeutika oder Farbstoffen. Insbesondere sind dabei Herbizide auf Cyclohexandionbasis zu nennen, wie sie z.B. in der DE-A-3 121 355 beschrieben sind.

Die nach dem erfindungsgemäßen Verfahren zugänglichen Verbindungen lassen sich z.B. leicht zu Aminen, Alkohlen und Säuren nach dem Fachmann geläufigen Methoden, z.B. durch Oxidation mit Sauerstoff oder durch Reduktion z.B. durch katalytische Hydrierung oder aminierende Hydrierung weiterverarbeiten, die ihrerseits wertvolle Zwischenprodukte darstellen.

Die folgenden Beispiele dienen zur weiteren Erläuterung der Erfindung.

Beispiel 1 - 34

Die Reaktionen werden unter isothermen Bedingungen in einem Rohrreaktor (Wendel 0,6 cm, 90 cm Länge) in der Gasphase mindestens 6 Stunden lang durchgeführt. Die Trennung und Charakterisierung der Reaktionsprodukte erfolgte nach üblichen Methoden. Die quantitative Bestimmung der Reaktionsprodukte und der Ausgangsstoffe erfolgte gaschromatographisch und durch CO-Zahl.

Die in den Beispielen für das erfindungsgemäße Verfahren verwendeten Katalysatoren sind:

Katalysator A

Der Aluminosilikatzeolith vom Pentasil-Typ wurde unter hydrothermalen Bedingungen bei autogenem Druck und 150°C aus 650 g hochdispersem $SiO_2$, 203 g $Al_2(SO_4)_3 \times 18\ H_2O$ in 10 kg einer wäßrigen 1,6-Hexandiamin-Lösung (Mischung 50:50 Gew.-%) in einem Rührautoklaven hergestellt. Nach Abfiltrieren und Auswaschen wurde das kristalline Reaktionsprodukt bei 110°C/24 h getrocknet und bei 500°C/24 h calciniert. Dieser Aluminosilikatzeolith enthielt 92,8 Gew.-% $SiO_2$ und 4,2 Gew.-% $Al_2O_3$.

Katalysator A wird erhalten, indem man den reinen Aluminosilikatzeolithen des Pentasil-Typs mit Verformungshilfsmitteln zu 2-mm-Strängen verformt, bei 110°C/16 h trocknet und bei 500°C/24 h calciniert.

Katalysator B

Der Borosilikatzeolith des Pentasil-Typs wird in einer hydrothermalen Synthese aus 640 g hochdispersem $SiO_2$, 122 g $H_3BO_3$, 8 kg einer wäßrigen 1,6-Hexandiamin-Lösung (Mischung 50 : 50 Gew.%) bei 170°C unter autogenem Druck in einem Rührautoklaven hergestellt. Nach Abfiltrieren und Auswaschen wird das kristalline Reaktionsprodukt bei 100°C/24 h getrocknet und bei 500°C/24 h calciniert. Dieser Borosilikatzeolith setzt sich zusammen aus 94,2 Gew.-% $SiO_2$ und 2,3 Gew.-% $B_2O_3$.

Mit diesem Material werden durch Verformen mit Verformungshilfsmitteln 2-mm-Stränge hergestellt, die bei 110°C/16 h getrocknet und bei 500°C/24 h calciniert werden.

Katalysator C

Katalysator C wird durch Imprägnieren des Katalysators B mit wäßriger $Cu(NO_3)_2$-Lösung erhalten. Nach abermaliger Trocknung und Calcination bei 540°C/2 h beträgt der Cu-Gehalt 3,4 Gew.-%.

Katalysator D

$AlPO_4$-9 (APO-9) wird synthetisiert, indem man 200 g 98 %ige Phosphorsäure und 136 g Boehmit in 400 g Wasser löst bzw. suspendiert, hierzu eine wäßrige Lösung aus 112 g Diazabicyclo-2,2,2-octan (DABCO) und 320 g $H_2O$ zugibt und diese Mischung in einem Rührautoklaven bei 200°C während 336 Std. unter autogenem Druck umsetzt. Nach Abfiltrieren wird das kristalline Material bei 120°C getrocknet und bei 500°C/16 h calciniert. Das so synthetisierte $AlPO_4$-9 enthält 49,0 Gew.-% $P_2O_5$, 37,1 Gew.-% $Al_2O_3$. Dieses Material wird mit Verstrangungshilfsmittel zu 3 mm Strängen verformt, abermals bei 120°C getrocknet und bei 500°C/6 h calciniert.

Katalysator E

Siliciumaluminiumphosphat-5 (SAPO-5) wird hergestellt aus einer Mischung aus 200 g 98 %iger Phosphorsäure, 136 g Boehmit, 60 g Kieselsol (30 %ig) 287 g Tripropylamin und 587 g $H_2O$. Diese Mischung wird bei 150°C während 168 Std. unter autogenem Druck umgesetzt. Nach Filtration wird das kristalline Produkt bei 120°C getrocknet und bei 500°C calciniert. SAPO-5 enthält 49,8 Gew.-% $P_2O_5$, 33,0 Gew.-% $Al_2O_3$, 6,2 Gew.-% $SiO_2$. SAPO-5 wird mit einem Verstrangungshilfsmittel zu 3 mm Strängen verformt, bei 120°C getrocknet und bei 500°C calciniert.

Katalysator F

$SiO_2$, im Handel erhältlich unter der Handelsbezeichnung D11-11 der Firma BASF-AG.

Die Versuchsergebnisse und Reaktionsparameter sind in den folgenden Tabellen zusammengestellt.

Tabelle 1

| Beispiel Nr. | 1 | 2 | 3 | 4 | 5 | 6 | 7 |
|---|---|---|---|---|---|---|---|
| Einsatzstoff der nachstehenden Formel | III' | III' | III' | IV' | IV' | V' | V' |
| Katalysator | A | B | F | B | F | A | B |
| Temperatur | 300°C | 300°C | 300°C | 300°C | 300°C | 300°C | 300°C |
| WHSV | 2 $h^{-1}$ | 2 $h^{-1}$ | 2 $h^{-1}$ | 3 $h^{-1}$ | 3 $h^{-1}$ | 2 $h^{-1}$ | 2 $h^{-1}$ |
| Umsatz % | 100 | 100 | 100 | 100 | 100 | 100 | 100 |
| Selektivität % | 97,6 | 96,6 | 96,5 | 96,0 | 93,0 | 91,8 | 91,6 |
| Ausbeute % 4-Formyl-tetrahydropyran | 97,6 | 96,6 | 96,5 | 96,0 | 93,0 | 91,8 | 91,6 |

III' = 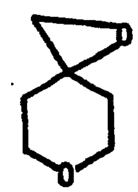 gelöst in THF 50 : 50 Gew.-%

IV' = 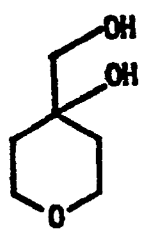 gelöst in THF 50 : 50 Gew.-%

V' = 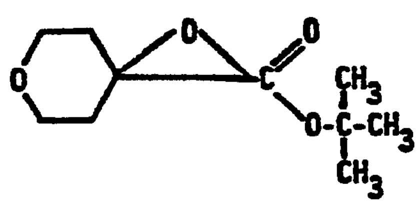 gelöst in THF 50 : 50 Gew.-%

Tabelle 2

| Beispiel Nr. | $R^1$ IIb ($R^4$ = H; $R^3$ + $R^2$ = –O–) | Katalysator | Ausbeute % an I | Siedepunkt °C/mbar |
|---|---|---|---|---|
| 8 | Ethyl | B | 59 | 80–82/22 |
| 9 | i-Propyl | B | 42 | |
| 10 | i-Propyl | F | 66 | 79–84/14 |
| 11 | i-Butyl | A | 72 [(50 + 22)[1]] | 44–46/0,1 |
| 12 | i-Propyl | B | 83 [(70 + 13)[1]] | 79–84/14 |
| 13 | i-Butyl | F | 76 [(56 + 20)[1]] | 44–46/01 |
| 14 | n-Octyl | A | 68 [(46 + 21)[1]] | |
| 15 | n-Octyl | C | 77 [(50 + 27)[1]] | – |
| 16 | Cyclododecyl | B | 67 (36 + 31)[1] | 130–140/0,01 |
| 17 | Methoxymethyl | B | 57 | |
| 18 | Methoxymethyl | F | 57 | 64–68/1,0 |
| 19 | 4-Tetrahydropyranyl | A | 56 | |
| 20 | 4-Tetrahydropyranyl | B | 51 | 172–180/0,5 |
| 21 | 4-Tetrahydropyranyl | F | 32 | |
| 22 | Phenyl | A | | |
| 23 | Phenyl | B | 76 (48 + 28)[1] | 102/0,1 |
| 24 | 4-Methoxyphenyl | A | 70 | |
| 25 | 4-Methoxyphenyl | B | | |
| 26 | 2-Chlorphenyl | A | | |
| 27 | 2-Chlorphenyl | B | 69 | |
| 28 | 3-Trifluormethylphenyl | B | 73 (50 + 23)[1] | 95–102/0,07 |
| 29 | 2-Furanyl | B | 49 | |
| 30 | 2-Furanyl | F | 41 | – |
| 31 | 3-Tetrahydropyranyl | B | 55 | |
| 32 | Benzyl | B | 62 | |
| 33 | 4-Fluorphenyl | B | 65 | |
| 34 | 4-Fluorphenyl | A | | |

[1] cis/trans-Isomere von I (nicht zugeordnet).

Vergleichsbeispiel 1

Zu einer Lösung von 81 g 2-Natriumcarboxyl-spiro-1,6-dioxa-[2,5]-octan in 500 ml Wasser werden während 4 Stunden bei 80 - 85°C 82,1 g 20 %ige wäßrige Salzsäure zugetropft, dann wird 30 min bei 85°C nachgerührt. Anschließend wird 3 Stunden wasserdampfdestilliert. Das erhaltene Destillat (ca. 2 Liter) wird mit Natriumchlorid gesättigt und siebenmal mit Methylenchlorid extrahiert. Die organische Phase wird über $Na_2SO_4$ getrocknet und das Lösungsmittel abdestilliert. Destillation des Rückstands ergibt 18,1 g (36 %) 4-Formyltetrahydropyran

Vergleichsbeispiel 2

Ein Gemisch aus 20 g Spiro-1,6-dioxa-[2,5]-octan und 0,3 g Zinkchlorid wird auf 250°C erwärmt. Während 1 Stunde werden 15,2 g einer Flüssigkeit abdestilliert, die gaschromatographisch ermittelt aus 6,7 g (33 %) Spiro-1,6-dioxa-[2,5]-octan und 8,5 g (42 %) 4-Formyltetrahydropyran besteht. Durch Destillation werden daraus 5,1 g 4-Formyltetrahydropyran erhalten. Beim Stehenlassen bei Raumtemperatur kristallisiert die zunächst klare Flüssigkeit zu einem farblosen Feststoff.

Das Ausgangsprodukt V′ wurde wie folgt hergestellt:

Zu einem Gemisch aus 179,5 g Chloressigsäure-tert.-butylester, 120 g 4-Oxotetrahydropyran und 1200 ml tert.-Butanol gibt man bei 10 - 15°C portionsweise 147,8 g Kalium-tert.-butylat zu und rührt 24

Stunden bei Raumtemperatur nach. Das Lösungsmittel wird abdestilliert und der Rückstand mit 500 ml Wasser versetzt. Dann wird mit Methyl-tert.-butylether extrahiert, die organische Phase über $Na_2SO_4$ getrocknet und eingeengt. Der Rückstand wird im Vakuum destilliert.
Siedepunkt: 74°C/0,2 mbar
Ausbeute: 210 g (81 % d.Th.) 2-tert.-Butoxycarbonyl-spiro-1,6-dioxa-[2,5]-octan

**Patentansprüche**

1. Verfahren zur Herstellung von 4-Formyltetrahydropyranen der allgemeinen Formel I

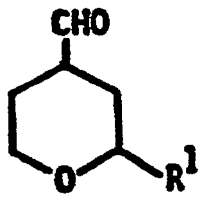

(I),

worin $R^1$ für Wasserstoff, $C_1$-$C_{12}$-Alkyl, $C_2$-$C_{12}$-Alkenyl, $C_1$-$C_6$-Alkoxy-substituiertes $C_1$-$C_{12}$-Alkyl, $C_4$-$C_{12}$-Cycloalkyl, gegebenenfalls substituiertes Aryl, Heteroaryl, oder Aralkyl, oder einen gegebenenfalls substituierten, gesättigten oder ungesättigten 5- oder 6-gliedrigen Ring, der gegebenenfalls durch N, O oder S unterbrochen ist, steht, daß dadurch gekennzeichnet ist, daß man
ein Pyranderivat der allgemeinen Formel II

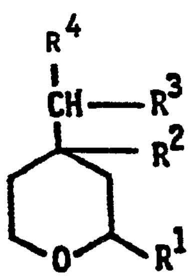

(II),

worin $R^1$ wie zuvor definiert ist,
$R^2$ und $R^3$ jeweils für eine OH-Gruppe stehen, oder gemeinsam ein Sauerstoffatom darstellen und dabei einen Oxiranring bilden, und
$R^4$ in dem Fall, daß $R^2$ und $R^3$ jeweils für eine OH-Gruppe stehen, Wasserstoff bedeutet und in dem Fall, daß $R^2$ und $R^3$ gemeinsam ein Sauerstoffatom darstellen, Wasserstoff oder tert.-Butyloxycarbonyl bedeutet,
bei erhöhter Temperatur mit einem Katalysator, ausgewählt unter Zeolithen, Phosphaten, Borsäure auf einem Trägermaterial und Siliciumdioxid, oder deren Mischungen, behandelt.

2. Verfahren nach Anspruch 1, dadurch gekennzeichnet, daß man als Ausgangsverbindung der allgemeinen Formel II 4-[1′,2′-Dihydroxy-ethyl]tetrahydropyran oder 1,6-Dioxaspiro[2,5]-octan einsetzt.

3. Verfahren nach einem der Ansprüche 1 oder 2, dadurch gekennzeichnet, daß man als Katalysator Zeolithe des Pentasil-Typs, Bor-, Eisen- oder Aluminiumsilicatzeolithe, Aluminiumsilicatzeolithe des Faujasit-, Mordenit- oder Erionit/Chabazit-Typs, oder mit Alkali- oder Erdalkalimetallen oder Übergangsmetallen oder Seltenerdmetallen dotierte Zeolithe, einsetzt.

4. Verfahren nach einem der Ansprüche 1 oder 2, dadurch gekennzeichnet, daß man als Katalysator gegebenenfalls hydrothermal hergestellte Phosphate der Elemente B, Al, Ce, Zr, Fe, Sr, oder deren Gemische, einsetzt.

5. Verfahren nach einem der Ansprüche 1, 2 und 4, dadurch gekennzeichnet, daß man als Katalysator hydrothermal hergestellte Aluminiumphosphate, Siliciumaluminiumphosphate oder Siliciumeisenaluminiumphosphate, einsetzt.

6. Verfahren nach einem der Ansprüche 1 oder 2, dadurch gekennzeichnet, daß man als Katalysator Phosphorsäure und/oder Borsäure auf Basis Siliciumdioxid oder Aluminiumoxid, einsetzt.

7. Verfahren nach einem der Ansprüche 1 bis 6, dadurch gekennzeichnet, daß man in der Gasphase arbeitet.

## Claims

1. A process for the preparation of a 4-formyltetrahydropyran of the formula I

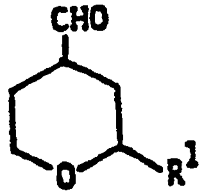

(I)

where $R^1$ is hydrogen, $C_1$–$C_{12}$-alkyl, $C_2$–$C_{12}$-alkenyl, $C_1$–$C_6$-alkoxy-substituted $C_1$–$C_{12}$-alkyl, $C_4$–$C_{12}$-cycloalkyl, unsubstituted or substituted aryl, hetaryl or aralkyl, or an unsubstituted or substituted, saturated or unsaturated 5-membered or 6-membered ring which may be interrupted by N, O or S, wherein a pyran derivative of the formula II

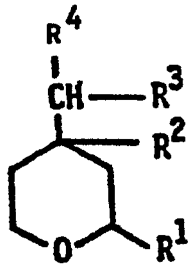

(II)

where $R^1$ is as defined above, $R^2$ and $R^3$ are each OH or together are an oxygen atom and thus form an oxirane ring, and $R^4$ is hydrogen where $R^2$ and $R^3$ are each OH, and is hydrogen or tert-butoxycarbonyl where $R^2$ and $R^3$ together are an oxygen atom, is treated at elevated temperatures with a catalyst selected from the group consisting of zeolites, phosphates, boric acid on a carrier and silica or mixtures of these.

2. A process as claimed in claim 1, wherein 4-[1′,2′-dihydroxyethyl]-tetrahydropyran or 1,6-dioxaspiro[2.5]-octane is used as the starting compound of the formula II.

3. A process as claimed in either of claims 1 and 2, wherein the catalyst used is a zeolite of the pentasil type, a borosilicate, iron silicate or aluminosilicate zeolite, an aluminosilicate zeolite of the faujasite, mordenite or erionite/chabazite type or a zeolite doped with alkali or alkaline earth metals or transition metals or rare earth metals.

4. A process as claimed in either of claims 1 and 2, wherein the catalyst used is a phosphate of B, Al, Ce, Zr, Fe or Sr or a mixture of these, which phosphate may have been prepared by a hydrothermal method.

5. A process as claimed in any of claims 1, 2 and 4, wherein the catalyst used is an aluminum phosphate, silicon aluminum phosphate or silicon iron aluminum phosphate prepared by a hydrothermal method.

6. A process as claimed in either of claims 1 and 2, wherein phosphoric acid and/or boric acid on silica or alumina are used as the catalyst.

7. A process as claimed in any of claims 1 to 6, which is carried out in the gas phase.

## Revendications

1. Procédé de préparation de 4-formyltétrahydropyrannes de formule générale I

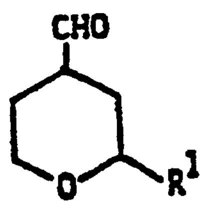

(I),

dans laquelle $R^1$ est mis pour un atome d'hydrogène, un groupement alkyle en $C_1$–$C_{12}$, alcényle en $C_2$–$C_{12}$, alkyle en $C_1$–$C_{12}$ substitué par un radical alcoxy en $C_1$–$C_6$, cycloalkyle en $C_4$–$C_{12}$, aryle, hétéroaryle ou aralkyle éventuellement substitué, ou un noyau à cinq ou six chaînons saturé ou insaturé éventuellement substitué, qui est éventuellement interrompu par N, O ou S, caractérisé en ce qu'on traite un dérivé de pyranne de formule générale II

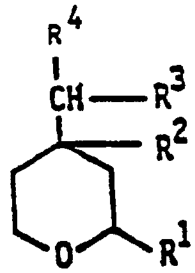

(II),

dans laquelle $R^1$ est tel que défini précédemment,
$R^2$ et $R^3$ sont chacun mis pour un groupement OH ou représentent ensemble un atome d'oxygène et forment ainsi un noyau oxiranne, et
$R^4$ dans le cas où $R^2$ et $R^3$ sont chacun mis pour un groupement OH représente un atome d'hydrogène et dans le cas où $R^2$ et $R^3$ représentent ensemble un atome d'oxygène, est mis pour un atome d'hydrogène ou un groupement tert.-butyloxycarbonyle, à températures élevées avec un catalyseur, choisi parmi les zéolites, les phosphates, les acides du bore sur un support et le bioxyde de silicium, ou leurs mélanges.

2. Procédé selon la revendication 1, caractérisé en ce qu'on utilise comme substances de départ de formule générale II le 4-[1',2'-dihydroxy-éthyl]tétrahydropyranne ou le 1,6-dioxaspiro[2,5]-octane.

3. Procédé selon l'une ou l'autre des revendications 1 et 2, caractérisé en ce qu'on utilise comme catalyseur des zéolites du type pentasil, des zéolites de boro-, ferro ou aluminosilicates, des zéolites d'alumino-silicates du type faujasite, mordénite, ou érionite/chabazite, ou des zéolites dopées par des métaux alcalins ou alcalino-terreux, des métaux de transition ou des métaux appartenant aux terres rares.

4. Procédé selon l'une ou l'autre des revendications 1 et 2, caractérisé en ce qu'on utilise comme catalyseur des phosphates éventuellement préparables par voie hydrothermique des éléments B, Al, Ce, Zr, Fe, Sr, ou de leurs mélanges.

5. Procédé selon l'une quelconque des revendications 1, 2 et 4, caractérisé en ce qu'on utilise comme catalyseur du phosphate d'aluminium, du phosphate de silicium et d'aluminium ou du phosphate de silicium, de fer et d'aluminium préparés par voie hydrothermique.

6. Procédé selon l'une ou l'autre des revendications 1 et 2, caractérisé en ce qu'on utilise comme catalyseur de l'acide phosphorique et/ou de l'acide borique sur une base de bioxyde de silicium ou d'oxyde d'aluminium.

7. Procédé selon l'une quelconque des revendications 1 à 6, caractérisé en ce qu'on travaille en phase gazeuse.